# EUROPEAN PATENT SPECIFICATION

(11) **EP 3 232 422 B1**
(45) Date of publication and mention of the grant of the patent: **26.06.2019**
(21) Application number: 14907678.8
(22) Date of filing: 09.12.2014
(51) Int. Cl.: G09B 23/38, C12M 1/00

(54) **PHOTOSYNTHESIS MICROFLUIDIC CHANNEL CHAMBER AND PHOTOSYNTHESIS METHOD**
PHOTOSYNTHESEKAMMER MIT MIKROFLUIDISCHEM KANAL UND PHOTOSYNTHESEVERFAHREN
CHAMBRE DE CANAL MICROFLUIDIQUE DE PHOTOSYNTHÈSE ET PROCÉDÉ DE PHOTOSYNTHÈSE

(43) Date of publication of application: 18.10.2017
(73) Proprietor: Lin, Po-Kang, Taipei City, Taiwan 104 (TW)
(72) Inventor: Lin, Po-Kang, Taipei City, Taiwan 104 (TW)
(74) Representative: Papula Oy
(86) International application number: PCT/CN2014/001112
(87) International publication number: WO 2016/090519

(56) References cited:
- WO-A1-2009/089185
- WO-A2-2008/042975
- CN-A- 102 224 079
- CN-A- 102 753 249
- CN-U- 203 673 740
- KR-A- 20120 110 262

## Description

### BACKGROUND OF THE INVENTION

### 1. Field of the Invention

The present invention relates generally to photosynthesis, more particularly to a photosynthetic device with a microfluid chamber and a method for causing photosynthesis in the microfluid chamber.

### 2. The Prior Arts

Owing to increase in concentration of so-called greenhouse gases (like carbon dioxide (CO₂), methan (CH₄) and nitrous oxide (N₂O)) produced by human activity and industry results in rising of the global temperature, which in turn, causes an increase evapotranspiration, thereby altering the heat balance system and changing the distribution of rainfall. That is causing the rainfall or floods in areas of drought originally, and the floods or rainfall in places where there is drought originally. Regarding the production of food crops, not only the temperature changes, so does the crop seasons, which is in need of moisture can not get water and/or vice versa, leading to significantly reducing the world crop production.

In addition to causing climate change, water crisis, food scarcity also result due to interactive effects. According to the UN assessment of global land resources, nearly a quarter of the world's agricultural land is affected or degraded seriously, but the world's population continues to grow. In order to feed all of humanity sufficiently, an increase of 70 per cent of food production should be increased by 2050 the latest year or else starvation will be prevalent in the near future.

As such, the real solution for the currently existing problems resides in how to solve the food shortage and how to effectively reduce emission of greenhouse gases lie ahead of us.

A patent publication WO 2009/089185 A1 discloses information that may be regarded as useful for understanding the background.

### SUMMARY OF THE INVENTION

It is an objective to provide a photosynthetic device with a micro fluid chamber for causing photosynthesis therein and method thereof. The object is achieved by the features of the independent claims. Some embodiments are described in the dependent claims.

A primary objective of the present invention is to provide a photosynthetic device with a micro fluid chamber for causing photosynthesis in the microfluid chamber. The photosynthetic device of the present invention includes a main body defining a microfluid chamber 100 for causing photosynthesis therein, and a light source, wherein the microfluid chamber is constituted by: at least one communication room, a plurality of micro channels respectively and spatially communicated with the communication room, at least one micro injection duct spatially communicated with the communication room, and a plurality of filter plugs spatially connected to the micro channels respectively and the micro injection duct at free ends thereof in order to filter fluid backflow in the micro channels and the micro injection duct. The light source radiates ceaselessly one of the communication room, the plurality of micro channels and the micro injection duct. The photosynthesis is resulted once chloroplasts and normal saline solution are injected into one of the communication room, the plurality of micro channels and the micro injection duct.

In the present invention, the normal saline solution is injected ceaselessly into one of the communication room, the plurality of micro channels and the micro injection duct while the filter plugs prevent the chloroplasts from spilling out therefrom.

In this embodiment, the micro channels and the micro injection duct are rotatable relative to the communication room.

The photosynthetic device of the present invention further includes an extra communication room and a connection micro channel interconnecting spatially the extra communication room with the communication room. Preferably, the connection micro channel is rotatable relative to the extra communication room and the communication room.

Another objective of the present invention is to provide a method for causing photosynthesis via a photosynthetic device. The method accordingly includes the steps: injecting chloroplasts and normal saline solution into a micro channel; ceaselessly injecting chloroplasts and normal saline solution into the micro channel; and radiating the micro channel simultaneously in order to cause photosynthesis within the micro channel; wherein, the photosynthetic device includes a main body defining a microfluid chamber for causing photosynthesis therein, the microfluid chamber is constituted by a communication room, a plurality of the micro channels respectively and spatially communicated with the communication room, at least one micro injection duct spatially communicated with the communication room, and a plurality of filter plugs spatially connected to the micro channels respectively and the micro injection duct at free ends thereof in order to filter fluid backflow in the micro channels and the micro injection duct.

The photosynthetic device of the present invention further includes an extra communication room and a connection micro channel interconnecting spatially the extra communication room with the communication room. Preferably, the connection micro channel is rotatable relative to the extra communication room and the communication room.

In addition, the micro channels and the micro injection duct are rotatable relative to the communication room.

### BRIEF DESCRIPTION OF THE DRAWINGS

The present invention will be apparent to those skilled in the art by reading the following detailed description of a preferred embodiment thereof, with reference to the attached drawings, in which:
Fig. 1 is a schematic diagram illustrating the first embodiment of a photosynthetic device of the present invention;
Fig. 2 is a schematic diagram illustrating the second embodiment of the photosynthetic device of the present invention;
Fig. 3 shows a block diagram illustrating the steps constituting a method for causing photosynthesis via a photosynthetic device of the present invention; and
Fig. 4 is a graph illustrating the relation between glucose amount vs photosynthesizing time carried out according to the photosynthesizing method of the present invention.

### DETAILED DESCRIPTION OF THE PREFERRED EMBODIMENT

The accompanying drawings are included to provide a further understanding of the invention, and are incorporated in and constitute a part of this specification. The drawings illustrate embodiments of the invention and, together with the description, serve to explain the principles of the invention.

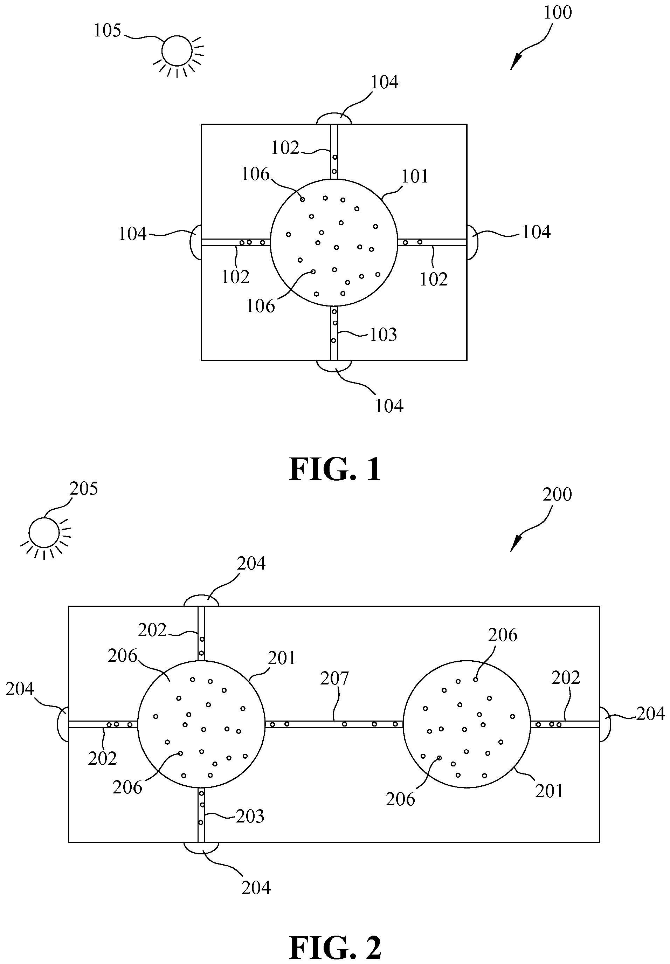

Fig. 1 is a schematic diagram illustrating the first embodiment of a photosynthetic device of the present invention. The photosynthetic device of the present invention includes a main body 100 and a light source 105. The main body 100 defines a micro fluid chamber for causing photosynthesis therein and preferably has a volume of 3.5cm^{∗}3.5cm^{∗}0.7cm, but the size thereof should not be limited only thereto. The micro fluid chamber is constituted by at least one communication room 101, a plurality of micro channels 102 respectively and spatially communicated with the communication room 101, at least one micro injection duct 103 spatially communicated with the communication room 101, and a plurality of filter plugs 104 spatially connected to the micro channels 102 respectively and the micro injection duct 103 at free ends thereof in order to filter fluid backflow in the aid micro channels 102 and the micro injection duct 103. The light source 105 is capable of radiating one of the communication room 101, the plurality of micro channels 102 and the micro injection duct 103. In this embodiment, the communication room 101, the micro channels 102 and the micro injection duct 103 are fabricated from transparent material, such as glass. The communication room 101 is circular in cross section and has a diameter of 2 cm, but the size should not be limited only thereto. Each of the micro channels 102 and the micro injection duct 103 has a first end connected spatially with the communication room 101 while the second ends of the micro channels 102 and the micro injection duct 103 are provided with the filter plugs 104 respectively such that photosynthesis is resulted once chloroplasts and normal saline solution are injected into one of the communication room 101, the micro channels 102 and the micro injection duct 103. Preferably, the micro channels 102 and the micro injection duct 103 are rotatable relative to the communication room 101 so as to evenly distribute the chloroplasts 106 within the channels and the injection duct in order to achieve the effective photosynthesis. Any connection means can be employed so long as it permits rotation of the micro channels 102 and the micro injection duct 103 relative to the communication room 101.

In this embodiment, the normal saline solution is injected ceaselessly into the communication room 101 and the micro channels 102 via the micro injection duct 103 while the filter plugs 104 prevent the chloroplasts from spilling out therefrom. Under this condition, chloroplasts within the normal saline solution are evenly and uniformly distributed so as to achieve the effective photosynthesis.

Fig. 2 is a schematic diagram illustrating the second embodiment of the photosynthetic device of the present invention;

The second embodiment is generally identical to the first embodiment in structure, except in that the second embodiment of the photosynthetic device of the present invention further includes an extra communication room 201 and a connection micro channel 207 interconnecting spatially the extra communication room 201 with the communication room 201. Preferably, the connection micro channel 207 is rotatable relative to the extra communication room 201 and the communication room 201 so as to distribute the chloroplasts 206 evenly and uniformly within the connection micro channel 207 in order to achieve the effective photosynthesis once chloroplasts and normal saline solution are ceaselessly injected via the micro injection duct 203. Since mounting and functions of the filter plugs 204 are identical to the first embodiment, a detailed description thereof is omitted for the sake of brevity. An important to note is that only a single micro injection duct 203 is used in the second embodiment, however the number should not be limited only thereto.

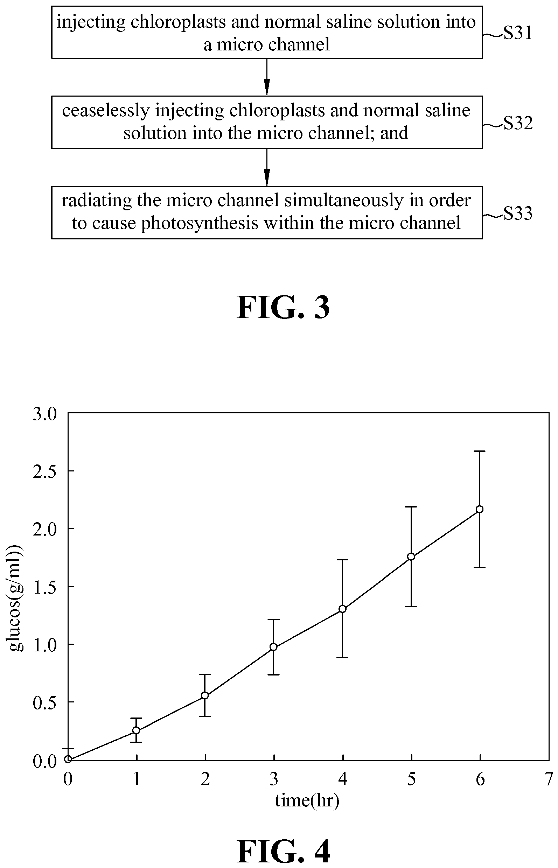

Fig. 3 shows a block diagram illustrating the steps constituting a method for causing photosynthesis via a photosynthetic device of the present invention.

The method for causing photosynthesis via a photosynthetic device, wherein, the photosynthetic device includes a main body defining a microfluid chamber for causing photosynthesis therein. The method accordingly includes the steps: S31 injecting chloroplasts and normal saline solution into a microfluid chamber for causing photosynthesis therein, the microfluid chamber is constituted by a communication room 202, a plurality of the micro channels 202 respectively and spatially communicated with the communication room 201, at least one micro injection duct 203 spatially communicated with the communication room 201, and a plurality of filter plugs 204 spatially connected to the micro channels 202 respectively and the micro injection duct 203 at free ends thereof in order to filter fluid backflow in the micro channels 202 and the micro injection duct 203. Preferably, the micro channels 102 and the micro injection duct 103 are rotatable relative to the communication room 101. In step S32, chloroplasts and normal saline solution are injected ceaselessly via the micro injection duct. In step S33, one of the micro channels is radiated by a light source (such as sunlight) in order to cause photosynthesis within the micro channel.

Fig. 4 is a graph illustrating the relation between glucose amount vs photosynthesizing time carried out according to the photosynthesizing method of the present invention.

As illustrated in Fig. 1, since the normal saline solution is injected ceaselessly into the communication room 101 and the micro channels 102 via the micro injection duct 103 so as to cause photosynthesis therein and after reaction glucose spills out from the filter plugs 104. As shown in Fig. 4, after one hour of photosynthesis, 0.25g/ml of glucose is obtained. After two hours of photosynthesis, 0.5g/ml of glucose is obtained while 2.0g/ml of glucose is obtained after 6 hours of photosynthesis.

One aspect to note is that the photosynthesis is caused not in the green plant, but rather in the micro fluid chamber of the present invention as long as we can extract chloroplasts from plants and using the same in the photosynthetic device of the present invention so as to produce the glucose in combination with water and sunlight. In other words, emission of carbon dioxide can be reduced when the photosynthetic device of the present invention is implemented.

Although the present invention has been described with reference to the preferred embodiments thereof, it is apparent to those skilled in the art that a variety of modifications and changes may be made without departing from the scope of the present invention which is intended to be defined by the appended claims.

## Claims

1. A photosynthetic device comprising:
a main body (100) defining a micro fluid chamber for causing photosynthesis therein, wherein the micro fluid chamber is constituted by:
at least one communication room (101),
a plurality of micro channels (102) respectively and spatially communicated with said communication room (101),
at least one micro injection duct (103) spatially communicated with said communication room (101), and
a plurality of filter plugs (104) spatially connected to said micro channels (102) respectively and said micro injection duct (103) at free ends thereof in order to filter fluid backflow in said micro channels (102) and said micro injection duct (103); and
a light source (105) for radiating one of said communication room (101), said plurality of micro channels (102) and said micro injection duct (103);
wherein, photosynthesis is resulted once chloroplasts and normal saline solution are injected into one of said communication room (101), said plurality of micro channels (102) and said micro injection duct (103); and
wherein said micro channels (102) and said micro injection duct (103) are rotatable relative to said communication room (101).

2. The photosynthetic device according to claim 1, wherein the normal saline solution is injected ceaselessly into one of said communication room (101), said plurality of micro channels (102) and said micro injection duct (103) while said filter plugs (104) prevent the chloroplasts from spilling out therefrom.

3. The photosynthetic device according to claim 1, further comprising an extra communication room (201) and a connection micro channel (207) interconnecting spatially said extra communication room (201) with said communication room (101).

4. The photosynthetic device according to claim 3, wherein said connection micro channel (207) is rotatable relative to said extra communication room (201) and said communication room (101).

5. A method for causing photosynthesis via a photosynthetic device comprising the steps:
injecting chloroplasts and normal saline solution into a micro channel (102);
ceaselessly injecting chloroplasts and normal saline solution into said micro channel (102); and
radiating said micro channel (102) simultaneously in order to cause photosynthesis within said micro channel (102);
wherein, the photosynthetic device includes a main body (100) defining a microfluid chamber for causing photosynthesis therein, said microfluid chamber is constituted by a communication room (101), a plurality of said micro channels (102) respectively and spatially communicated with said communication room (101), at least one micro injection duct (103) spatially communicated with said communication room (101), and a plurality of filter plugs (104) spatially connected to said micro channels (102) respectively and said micro injection duct (103) at free ends thereof in order to filter fluid backflow in said micro channels (102) and said micro injection duct (103); and
wherein said micro channels (102) and said micro injection duct (103) are rotatable relative to said communication room (101).

6. The method according to claim 5, wherein the photosynthetic device further includes a light source (105) for radiating ceaselessly to one of said communication room (101), said plurality of micro channels (102) and said micro injection duct (103), an extra communication room (201) and a connection micro channel (207) interconnecting spatially said extra communication room (201) with said communication room (101).

7. The method according to claim 6, wherein said connection micro channel (207) is rotatable relative to said extra communication room (201) and said communication room (101).

## Patentansprüche

1. Photosynthesevorrichtung, die aufweist:
einen Hauptkörper (100), der eine Mikrofluidkammer zum Bewirken von Photosynthese in derselben definiert, wobei die Mikrofluidkammer gebildet ist durch:
mindestens einen Kommunikationsraum (101),
mehrere Mikrokanäle (102), die jeweils und räumlich mit dem Kommunikationsraum (101) in Verbindung stehen,
mindestens eine Mikroeinspritzleitung (103), die räumlich mit dem Kommunikationsraum (101) in Verbindung steht, und
mehrere Filterstopfen (104), die jeweils räumlich mit den Mikrokanälen (102) und der Mikroeinspritzleitung (103) an freien Enden derselben verbunden sind, um Fluidrückfluss in den Mikrokanälen (102) und der Mikroeinspritzleitung (103) zu filtern; und
eine Lichtquelle (105) zum Bestrahlen von einem von dem Kommunikationsraum (101), den mehreren Mikrokanälen (102) und der Mikroeinspritzleitung (103);
wobei Photosynthese hervorgerufen wird, wenn Chloroplasten und normale Kochsalzlösung in eines von dem Kommunikationsraum (101), den mehreren Mikrokanälen (102) und der Mikroeinspritzleitung (103) eingespritzt sind; und
wobei die Mikrokanäle (102) und die Mikroeinspritzleitung (103) relativ zu dem Kommunikationsraum (101) drehbar sind.

2. Photosynthesevorrichtung gemäß Anspruch 1, bei der die normale Kochsalzlösung ständig in eines von dem Kommunikationsraum (101), den mehreren Mikrokanälen (102) und der Mikroeinspritzleitung (103) eingespritzt ist, während die Filterstopfen (104) ein Austreten der Chloroplasten daraus verhindern.

3. Photosynthesevorrichtung gemäß Anspruch 1, die ferner einen zusätzlichen Kommunikationsraum (201) und einen Verbindungsmikrokanal (207), der den zusätzlichen Kommunikationsraum (201) räumlich mit dem Kommunikationsraum (101) verbindet, aufweist.

4. Photosynthesevorrichtung gemäß Anspruch 3, bei der der Verbindungsmikrokanal (207) relativ zu dem zusätzlichen Kommunikationsraum (201) und dem Kommunikationsraum (101) drehbar ist.

5. Verfahren zum Bewirken von Photosynthese über eine Photosynthesevorrichtung, mit den Schritten:
Einspritzen von Chloroplasten und normaler Kochsalzlösung in einen Mikrokanal (102);
ständiges Einspritzen von Chloroplasten und normaler Kochsalzlösung in den Mikrokanal (102) und
gleichzeitiges Bestrahlen des Mikrokanals (102), um Photosynthese in dem Mikrokanal (102) zu bewirken;
wobei die Photosynthesevorrichtung einen Hauptkörper (100) umfasst, der eine Mikrofluidkammer zum Bewirken von Photosynthese in derselben umfasst, wobei die Mikrofluidkammer gebildet ist durch einen Kommunikationsraum (101), mehrere der Mikrokanäle (102), die jeweils und räumlich mit dem Kommunikationsraum (101) in Verbindung stehen, mindestens eine Mikroeinspritzleitung (103), die räumlich mit dem Kommunikationsraum (101) in Verbindung steht, und mehrere Filterstopfen (104), die jeweils räumlich mit den Mikrokanälen (102) und der Mikroeinspritzleitung (103) an freien Enden derselben verbunden sind, um Fluidrückfluss in den Mikrokanälen (102) und der Mikroeinspritzleitung (103) zu filtern; und
wobei die Mikrokanäle (102) und die Mikroeinspritzleitung (103) relativ zu dem Kommunikationsraum (101) drehbar sind.

6. Verfahren gemäß Anspruch 5, bei dem die Photosynthesevorrichtung ferner eine Lichtquelle (105) zum ständigen Strahlen zu einem von dem Kommunikationsraum (101), den mehreren Mikrokanälen (102) und der Mikroeinspritzleitung (103), einen zusätzlichen Kommunikationsraum (201) und einen Verbindungsmikrokanal (207), der den zusätzlichen Kommunikationsraum (201) räumlich mit dem Kommunikationsraum (101) verbindet, umfasst.

7. Verfahren gemäß Anspruch 6, bei dem der Verbindungsmikrokanal (207) relativ zu dem zusätzlichen Kommunikationsraum (201) und dem Kommunikationsraum (101) drehbar ist.

## Revendications

1. Dispositif photosynthétique comprenant :
un corps principal (100) définissant une chambre de microfluide pour y effectuer une photosynthèse, dans lequel la chambre de microfluide est constituée par :
au moins un espace de communication (101),
une pluralité de microcanaux (102) respectivement en communication spatiale avec ledit espace de communication (101),
au moins un microconduit d'injection (103) en communication spatiale avec ledit espace de communication (101) et
une pluralité de bouchons de filtre (104) raccordés spatialement auxdits microcanaux (102) respectivement et audit microconduit d'injection (103) à leurs extrémités libres afin de filtrer le reflux de fluide dans lesdits microcanaux (102) et ledit microconduit d'injection (103) ; et
une source de lumière (105) pour irradier l'un(e) dudit espace de communication (101), de ladite pluralité de microcanaux (102) et dudit microconduit d'injection (103) ;
dans lequel la photosynthèse est obtenue une fois que des chloroplastes et une solution saline normale sont injectés dans l'un(e) dudit espace de communication (101), de ladite pluralité de microcanaux (102) et dudit microconduit d'injection (103) ; et
dans lequel lesdits microcanaux (102) et ledit microconduit d'injection (103) peuvent tourner par rapport audit espace de communication (101).

2. Dispositif photosynthétique selon la revendication 1, dans lequel la solution saline normale est injectée en permanence dans l'un (e) dudit espace de communication (101), de ladite pluralité de microcanaux (102) et dudit microconduit d'injection (103) tandis que lesdits bouchons de filtre (104) empêchent les chloroplastes de s'en répandre.

3. Dispositif photosynthétique selon la revendication 1, comprenant en outre un espace de communication supplémentaire (201) et un microcanal de jonction (207) interconnectant spatialement ledit espace de communication supplémentaire (201) avec ledit espace de communication (101).

4. Dispositif photosynthétique selon la revendication 3, dans lequel ledit microcanal de jonction (207) peut tourner par rapport audit espace de communication supplémentaire (201) et audit espace de communication (101).

5. Procédé pour provoquer une photosynthèse via un dispositif photosynthétique, comprenant les étapes consistant à :
injecter des chloroplastes et une solution saline normale dans un microcanal (102) ;
injecter en permanence des chloroplastes et une solution saline normale dans ledit microcanal (102) ; et
irradier ledit microcanal (102) simultanément afin de provoquer une photosynthèse dans ledit microcanal (102) ;
dans lequel le dispositif photosynthétique inclut un corps principal (100) définissant une chambre de microfluide pour y effectuer une photosynthèse, ladite chambre de microfluide est constituée par un espace de communication (101), une pluralité desdits microcanaux (102) respectivement en communication spatiale avec ledit espace de communication (101), au moins un microconduit d'injection (103) en communication spatiale avec ledit espace de communication (101) et une pluralité de bouchons de filtre (104) raccordés spatialement auxdits microcanaux (102), respectivement, et ledit microconduit d'injection(103) à leurs extrémités libres afin de filtrer le reflux de fluide dans lesdits microcanaux (102) et ledit microconduit d'injection (103) ; et
dans lequel lesdits microcanaux (102) et ledit microconduit d'injection (103) peuvent tourner par rapport audit espace de communication (101).

6. Procédé selon la revendication 5, dans lequel le dispositif photosynthétique comprend en outre une source de lumière (105) pour irradier en permanence l'un(e) dudit espace de communication (101), de ladite pluralité de microcanaux (102) et dudit microconduit d'injection (103), un espace de communication supplémentaire (201) et un microcanal de jonction (207) interconnectant spatialement ledit espace de communication supplémentaire (201) audit espace de communication (101).

7. Procédé selon la revendication 6, dans ledit microcanal de jonction (207) peut tourner par rapport audit espace de communication supplémentaire (201) et audit espace de communication (101).
